# EUROPEAN PATENT APPLICATION

(11) **EP 4 368 129 A1**
(43) Date of publication of application: **15.05.2024**
(21) Application number: 23201967.9
(22) Date of filing: 05.10.2023
(51) Int. Cl.: A61B 17/54, A61B 17/225, A61N 7/00, A61N 5/06, A61M 5/155, A61M 37/00, A61H 9/00, A61H 23/00

(54) **A MULTIFUNCTIONAL COSMETIC DEVICE AND A MEANS OF GUIDING BY PRESENTING ANIMATED INFORMATION WHICH ENABLES SELF-HANDLING**

(30) Priority: 22.10.2022 PL 44261322
(71) Applicant: Matuszak, Michal, 63-460 Maczniki (PL)
(72) Inventor: Matuszak, Michal, 63-460 Maczniki (PL)
(74) Representative: Jedrzejewski, Michal

(57) **Abstract**

A multifunctional cosmetic device for rejuvenating and body shaping treatments, characteristic for its compact size in relation to large professional devices and intended for use at home; it consists of a base station (1) for controlling, connecting and starting respective heads, a shockwave compressor head (2) for body shaping and firming, which operates under the compressor pressure, a LED head (3) for light therapy, a head (4a) for endermomassage with a diameter of at least 50 mm and a head (4b) with a diameter of at least 90 mm, a head (5) for oxygen infusion, that is oxybrasion, a head (6) for nano-needle mesotherapy, a head (7) for diamond microdermabrasion and a head (8) for oxygen infusion; the device is controlled by means of the base station (1) equipped with a touch screen (1a), and software and algorithms for treatment programmes or single treatments for a face, a neck, a cleavage, arms, a stomach, bottoms, thighs, palms, feet and a head skin with a "step by step" guiding mode. After creating a USER PROFILE 21 and entering CONTRAINDICATIONS 9 by means of information presented in the form of a text and animation, the device provides instructions which enable the user, staying at home, to perform independently only the treatments or treatment programmes which are safe for such a user.
Guiding treatments or treatment programmes by means of a multifunctional touch screen of the cosmetic device is characteristic for the fact that after creating and saving a USER PROFILE 21 and entering CONTRAINDICATIONS 9, a SELECTION SCREEN 10 is displayed with an option of choosing one of three operating modes: TREATMENTS 11 with access to possible single treatments for a given part of the body, which take into account CONTRAINDICATIONS 9; PROGRAMMES 12 with access to possible treatment programmes for a given part of the body which take into account CONTRAINDICATIONS 9; and PRO 13 with access to all the possible treatments but with indication for use by professionals.

## Description

The subject matter of the invention is a multifunctional cosmetic device and a means of guiding by presenting animated information on the device screen which, owing to the special presentation and specific guiding, enables people without medical knowledge, in particular, without cosmetic know-how, to conduct many cosmetic treatments at home.

Diamond microdermabrasion uses abrasive heads coated with diamond and under-pressure generated by a compressor installed in the machine. Diamond microdermabrasion consists in the controlled exfoliation of dead epidermis which, by means of under-pressure, migrates to a special filter.

Oxybrasion is an innovative treatment with a double-phase stream containing the micro-drops of physiological saline and oxygen. Since it does not cause any post-treatment irritations, oxybrasion is called "soft" dermabrasion. Oxybrasion not only has a peeling effect but it also moisturises and oxygenates the skin.

Endermomassage is a non-invasive vacuum therapy method combined with the effect of self-propelled rollers and a bipolar radio wave. The treatment with this device is completely non-invasive and gives excellent results in fighting against cellulite. It boosts metabolism up, improves tissue nutrition, reduces fat tissue and shapes the body. Sucking a skin fold expands blood vessels, enhances metabolism and, in consequence, the rate of eliminating toxins from cells. Lymph is discharged by the movement of the treatment head rollers. A bipolar radio wave has a direct effect on the skin and subcutaneous tissues. It heats the tissues up to 60°C and constricts collagen fibres. Skin becomes more elastic and definitely denser.

Mesotherapy is a method of forcing substances inside the skin. There is no-needle mesotherapy and needle mesotherapy. The most common devices for no-needle mesotherapy make use of an electroporation phenomenon. An impulse electromagnetic wave reaches the cells and supplies substances which are placed in a dispenser of a mesotherapy device. A no-needle mesotherapy is a wonderful alternative to needle treatments and it does not break the epidermal and skin integrity. Apparatuses for micro-needle mesotherapy are very popular. The devices of this type are equipped with a cartridge with several dozen needles used for micro insertions. Owing to this treatment, a selected serum may be applied to the skin at an insertion depth we choose. The treatments of this method are very popular and appreciated worldwide. 2in1 cosmetic combines are very common; they are equipped with micro-needle mesotherapy and permanent makeup. This treatment is mainly used in all the rejuvenating, slimming and anti-cellulite therapies. Nano-needle mesotherapy uses highfrequency strokes aimed at introducing active substances deep into the epidermis. The head has a cartridge containing 0.1 mm-long nano-needles which are used to apply gently the chosen ampoule.

LED is a light treatment with different wavelengths. It is a non-invasive and safe method that does not break the epidermis integrity. This technology uses white, blue, green, red, orange and yellow light. Each of them has a different effect, i.a.: micro blood circulation stimulation, anti-inflammatory, antibacterial and anti-seborrheic effect, exfoliation process regulation, would healing acceleration.

Multifunction cosmetic devices, also known as "combines", are devices with a few or even several functions which may be used by a beautician to conduct a treatment or even a whole therapy. The most demanded functions in such a device are: diamond microdermabrasion, dermo-massage, cavitation peeling (with additional functions: iontophoresis, galvanic skin treatment, electrostimulation, LED light therapy, ultrasounds, mesotherapy. Yet, a set of devices which will be included in a given "combine" depends on a customer who will configure or purchase such a device. A person configuring the device is usually a beautician who wants to equip her salon with specific devices to perform particular treatments or therapies. However, most of such treatments should be performed in a professional salon and under the supervision of an expert who is able to recognise contraindications and apply a treatment or therapy depending on expectations and possibilities.

The subject matter of the invention was to construct a device which would allow for performing many cosmetic body and face treatments at home by people without expertise. By means of ready treatment programmes and algorithms, a person without expertise, guided "step by step" through respective treatment stages or the entire therapy, has a possibility of benefiting from treatment cycles without leaving home, safely and comfortably.

The cosmetic combine, according to the invention, is a monolithic device for a single face or body treatment, or a comprehensive therapy, taking into account contraindications which block treatments that are forbidden or advised against in a specific case, and all this is possible thanks to special guiding and presentation on the screen by means of animation and simple information.

The essence of the invention, according to the application, is a multifunctional cosmetic device for rejuvenating and body shaping treatments, characteristic for its compact size in relation to large professional devices and intended for use at home; it consists of a base station for controlling, connecting and starting respective heads, a shockwave compressor head for body shaping and firming, which operates under the compressor pressure, a LED head for light therapy, a head for endermomassage with a diameter of at least 60 mm and a head with a diameter of at least 100 mm, a head for oxygen infusion, that is oxybrasion, a head for nano-needle mesotherapy, a head for diamond microdermabrasion and a head for oxygen infusion; whereas, the device is controlled by means of the base station equipped with a touch screen, and software and algorithms for treatment programmes or single treatments for a face, a neck, a cleavage, arms, a stomach, bottoms, thighs, palms, feet and a head skin with a "step by step" guiding mode. After creating a USER PROFILE and entering CONTRAINDICATIONS by means of information presented in the form of a text and animation, the device provides instructions which enable the user, staying at home, to perform independently only the treatments or treatment programmes which are safe for such a user.

Guiding treatments or treatment programmes by means of a multifunctional touch screen of the cosmetic device is characteristic for the fact that after creating and saving a USER PROFILE and entering CONTRAINDICATIONS, a SELECTION SCREEN is displayed with an option of choosing one of three operating modes: TREATMENTS with access to possible single treatments for a given part of the body, which take into account CONTRAINDICATIONS; PROGRAMMES with access to possible treatment programmes for a given part of the body which take into account CONTRAINDICATIONS; and PRO with access to all the possible treatments but with indication for use by professionals. Moreover, the device has a system of MESSAGES AND WARNINGS in case a user wants to undergo treatments advised against or treatments in which a user selects excessive time or increased dose. In addition, after each finished treatment, the device displays the RECOMMENDATIONS of behaviours after a given treatment and suggestions of transferring some treatments to a group of preferred treatments, that is FAVOURITES.

In one of the examples of guiding a treatment after selecting the FACE icon in the PROGRAMMES operating mode, a possibility of choosing various treatment programmes is displayed, and after choosing one of them, for instance, SCARS, the device proposes a diamond Microdermabrasion and/or nano-needle Mesotherapy and/or orange LED therapy, then a board is displayed with products and heads necessary for performing the treatment. After choosing the diamond Microdermabrasion treatment, the device informs on the subsequent board about ingredients which should be found in the preparation for diamond Microdermabrasion, and the following board provides general information to the user concerning the DIAMOND MICRODERMABRASION. Then, in the form of a short animated film and a description, the device demonstrates subsequent activities which must be done to prepare your skin for the treatment, to prepare the device for work and it shows how to apply the head and how to move it on the skin in order to perform the treatment effectively and correctly, and afterwards, how to dismantle and clean the head so that it is ready for work next time. After finishing the treatment and cleaning the head, the device presents the RECOMMENDATIONS of behaviours after the treatment.

In one of the examples of guiding a treatment after selecting the FACE icon in the PROGRAMMES operating mode, a possibility of choosing various treatment programmes is displayed, and after choosing one of them, for instance, SCARS, the device proposes a diamond Microdermabrasion and/or nano-needle Mesotherapy and/or orange LED therapy, then a board is displayed with products and heads necessary for performing the treatment. After selecting the nano-needle Mesotherapy treatment and displaying general information about this type of treatment, on the subsequent board, the device informs about the way of cleaning and preparing your face skin for the treatment, and afterwards the device instructs to apply a preparation for the nano-needle mesotherapy and specifies ingredients which should be found in a preparation for Mesotherapy. Then, the device instructs to wear protective gloves and, by means of a short animated film and a description, it demonstrates subsequent activities which must be done to prepare the device for work by selecting a proper cartridge, and it informs that the mesotherapy head has an option of adjusting the extension slide, suggesting a proper extension slide for respective parts of the face. The device informs how to set a proper extension slide and how to apply the head, and how to move it on the skin in order to perform the treatment effectively and correctly, and afterwards, how to dismantle and clean the head so that it is ready for work next time. After finishing the treatment and cleaning the head, the device presents the RECOMMENDATIONS of behaviours after the treatment.

In one of the examples of guiding a treatment after selecting the FACE icon in the PROGRAMMES operating mode, a possibility of choosing various treatment programmes is displayed, and after choosing one of them, for instance, SCARS, the device proposes a diamond Microdermabrasion and/or nano-needle Mesotherapy and/or orange LED therapy, then a board is displayed with products and heads necessary for performing the treatment. After selecting the orange LED treatment and displaying general information about this type of treatment, on the subsequent board, the device informs about the way of cleaning and preparing your face skin for the treatment, and afterwards the device instructs to apply a preparation for the LED head treatment. Then, the device informs how to prepare it and how to move the head on the skin in order to perform the treatment effectively and correctly, and afterwards it instructs to use a moisturising mask on the face and how to dismantle and clean the head so that it is ready for work next time. After finishing the treatment and cleaning the head, the device displays the RECOMMENDATIONS of behaviours after the treatment.

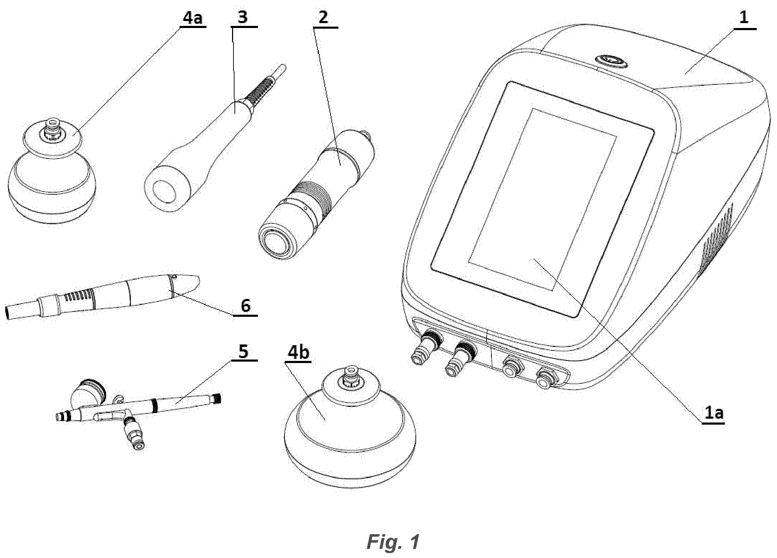

The subject matter of the invention is presented and clarified in the example of performance in the figure, in which
fig. 1 presents a base station 1 and a touch screen 1a, and a set of heads which are delivered with the device,
fig. 21 presents a screen image for creating a USER PROFILE 21,

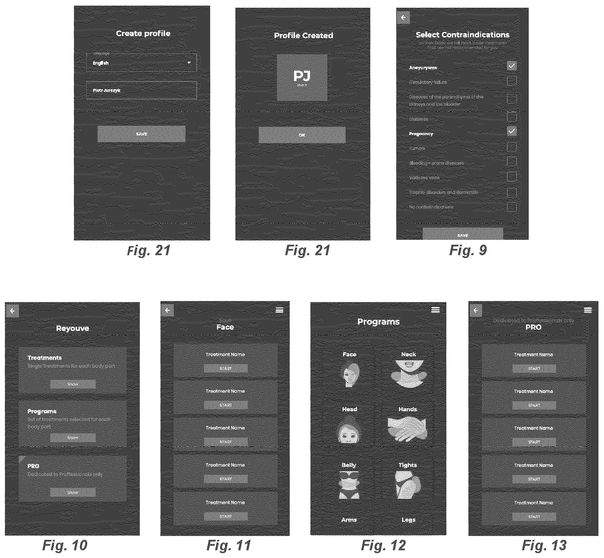

fig. 9 presents a screen image with CONTRAINDICATIONS 9,
fig. 10 presents a screen image with the SELECTION SCREEN 10,
fig. 11 presents a screen image with TREATMENTS selection 11,
fig. 12 presents a screen image with the PROGRAMMES selection 12,
fig. 13 presents a screen image with PRO 13,

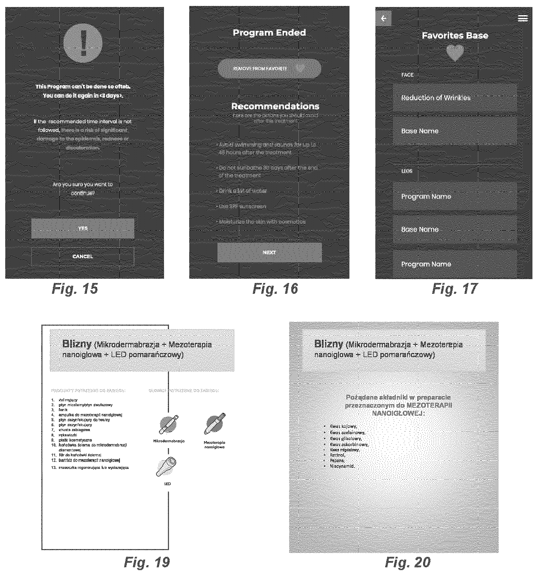

fig. 15 presents a screen image with MESSAGES AND WARNINGS 15,

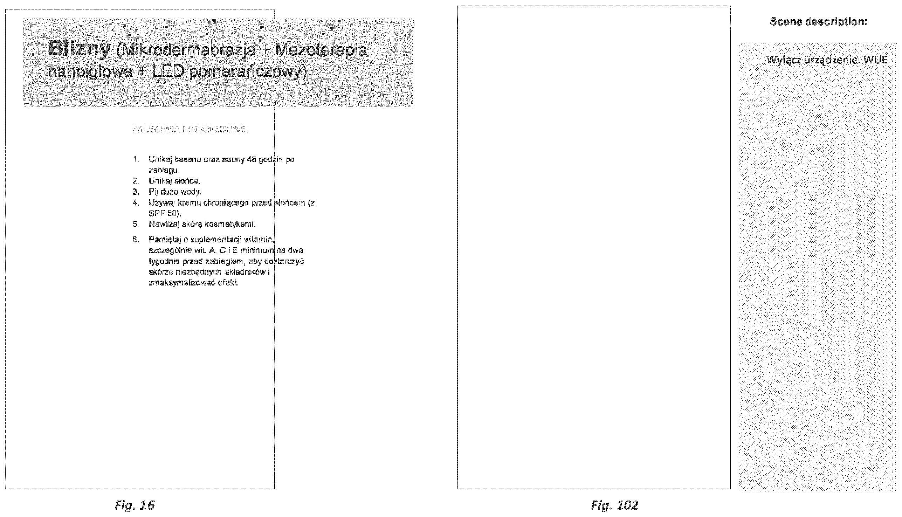

fig. 16 presents a screen image with RECOMMENDATIONS 16 after a finished treatment,
fig. 17 presents a screen image with FAVOURITES 17,
fig. 19 presents a screen image with a board 19 with products and heads necessary for performing a given treatment,
fig. 20 presents a screen image with a board 20 with ingredients which should be found in a preparation for a given treatment,

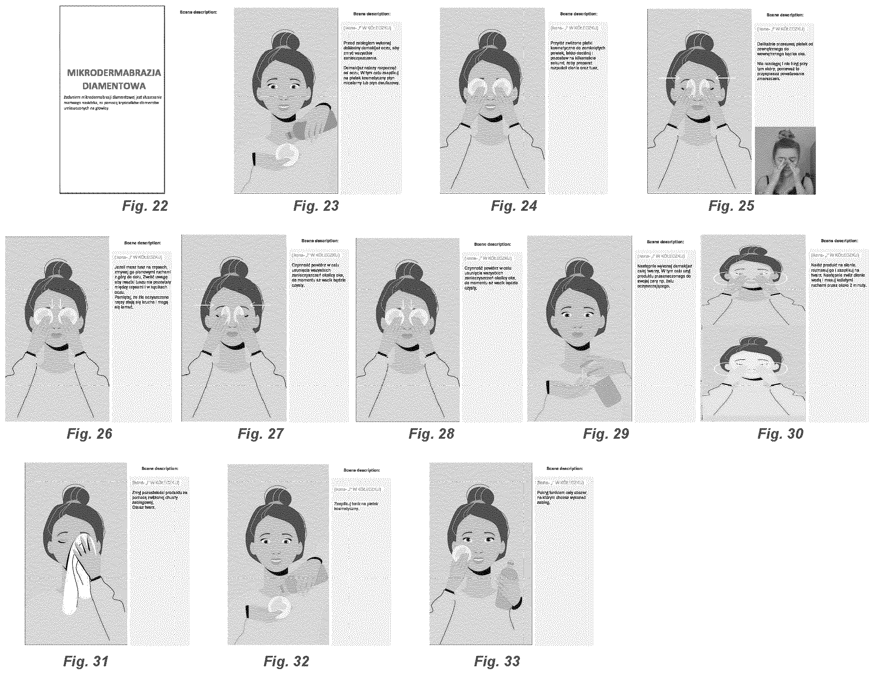

fig. 22 presents a screen image with board 22 with general information about a selected treatment,
fig. 23 - 33 present subsequent images with an animated film illustrating the way of preparing a face for a given treatment,

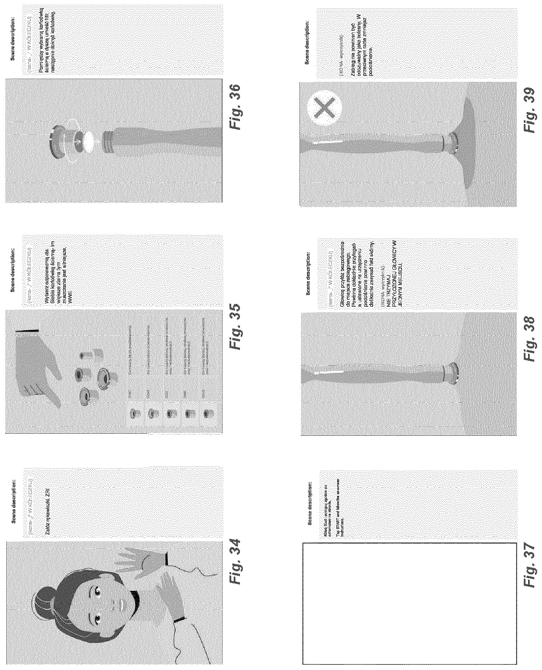

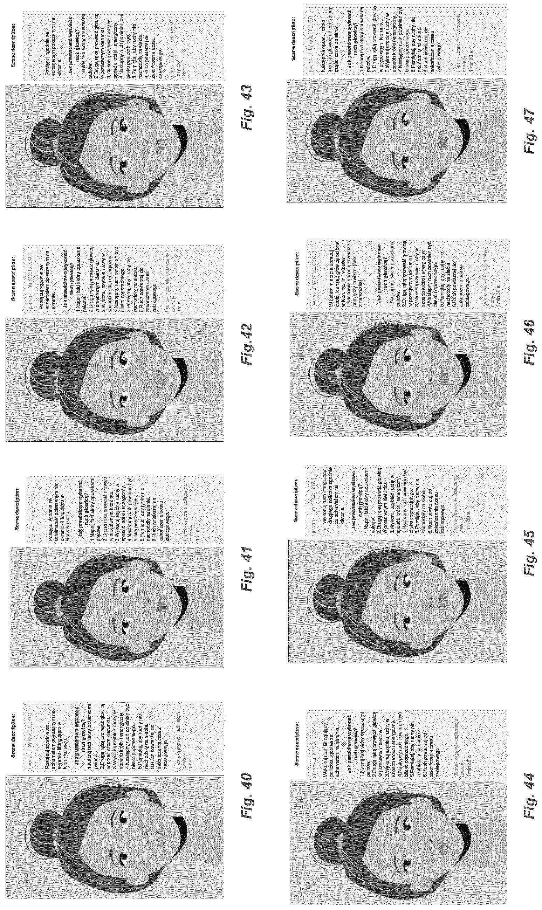

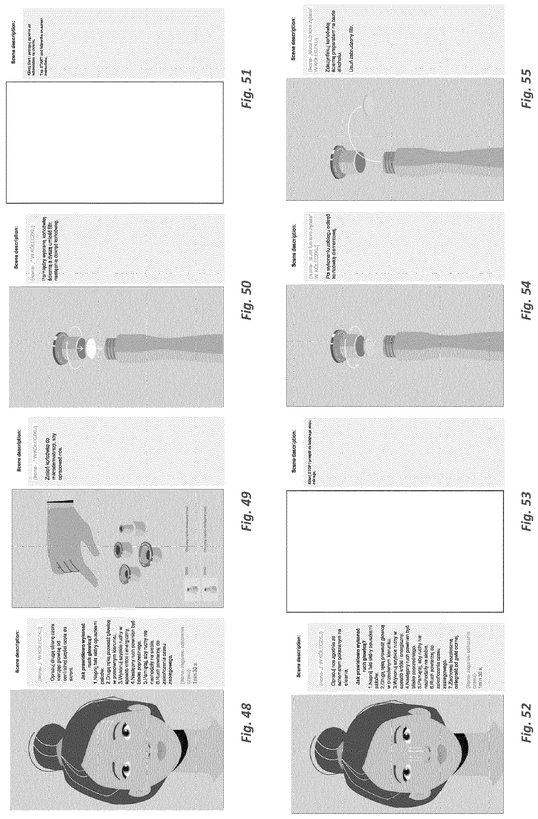

fig. 34 - 55 present subsequent images with an animated film illustrating the way of preparing the head, using a selected head and cleaning the head after the treatment,

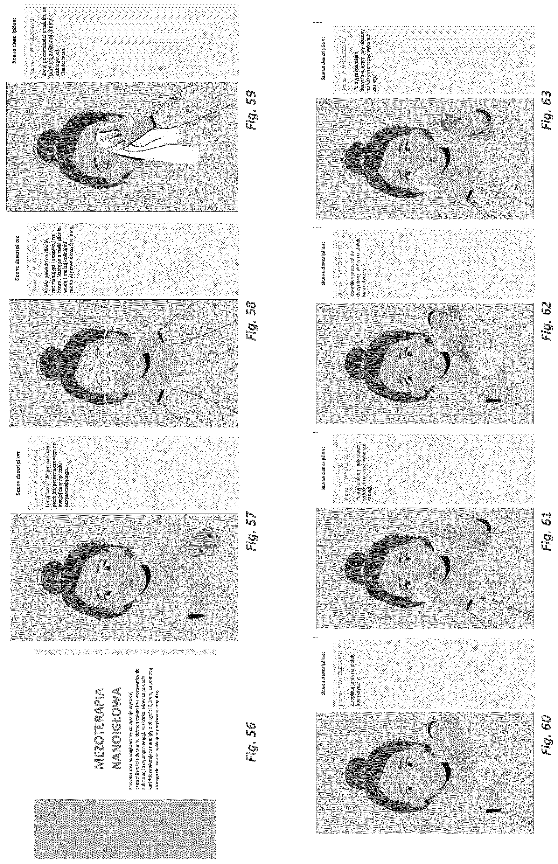

fig. 56 presents a screen image with a board with general information about a selected treatment,
fig. 57 - 63 present subsequent images with an animated film illustrating the way of preparing a face for a given treatment,

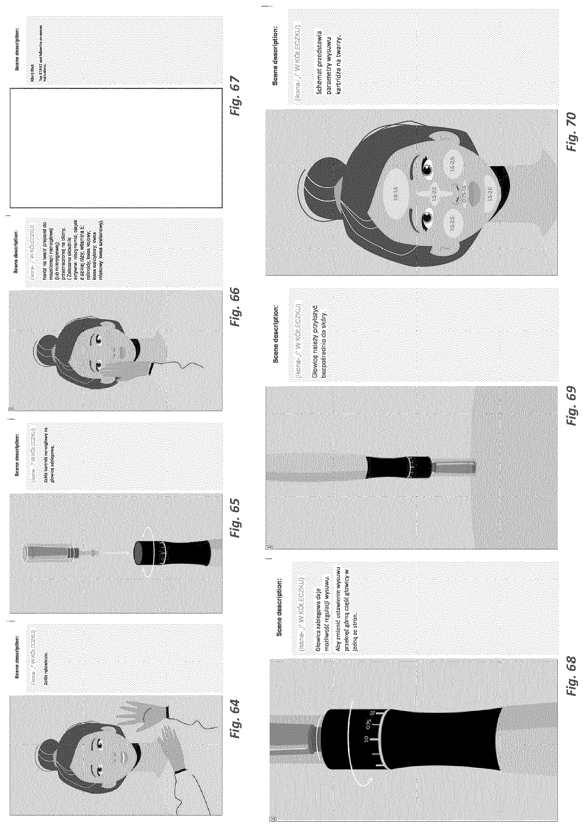

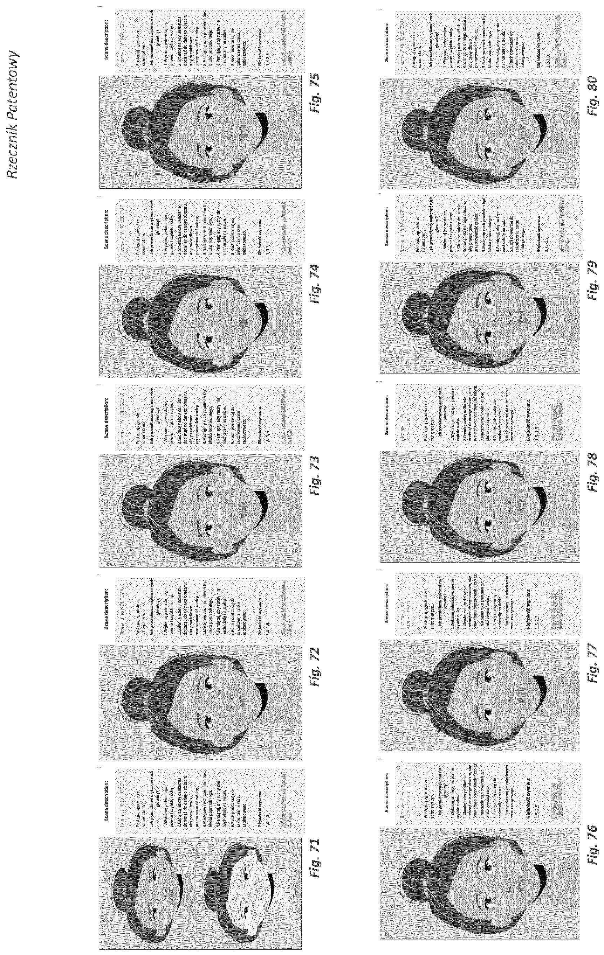

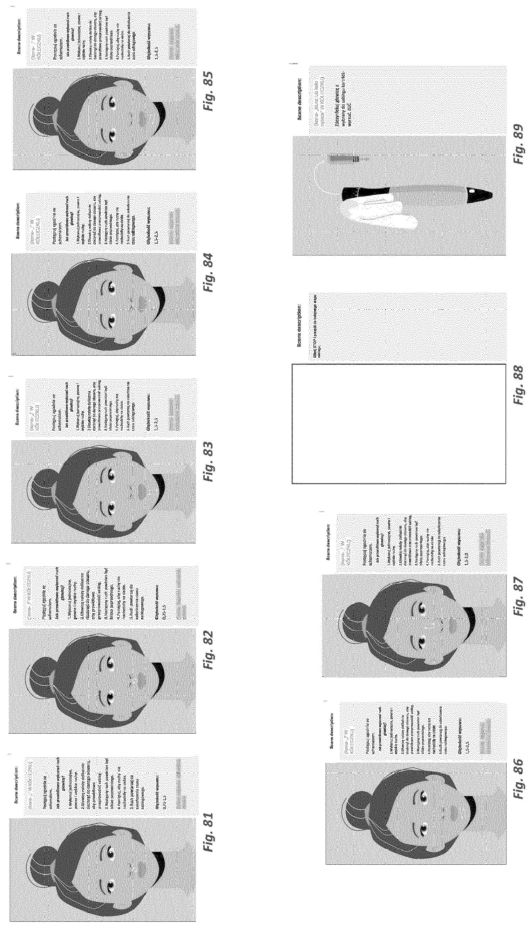

fig. 64 - 89 present subsequent images with an animated film illustrating the way of preparing the head, using a selected head, including the presentation of face zones, and cleaning the head after the treatment,

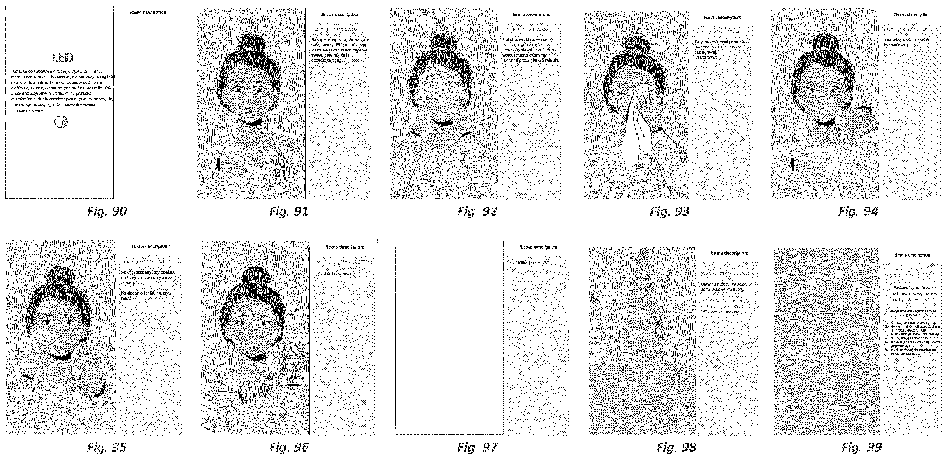

fig. 90 presents a screen image with a board with general information about a selected treatment,
fig. 91 - 95 present subsequent images with an animated film illustrating the way of preparing a face for a given treatment,

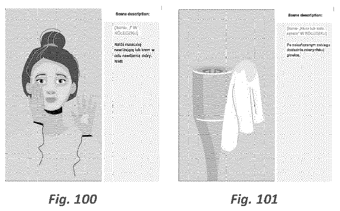

fig. 96 - 101 present subsequent images with an animated film illustrating the way of applying and using the head, including the presentation of movements which should be performed and face care, and the way of cleaning the head after the treatment,
fig. 16 presents a screen image with RECOMMENDATIONS 16 after a finished treatment,
fig. 102 presents final information about the required deactivation of the device.

A multifunctional cosmetic device for rejuvenating and body shaping treatments, characteristic for its compact size in relation to large professional devices, which are available on the market, and intended for use at home. The device, according to the invention, consists of a base station 1 for controlling, connecting and starting respective heads and a set of heads: a shockwave compressor head 2 for body shaping and firming, which operates under the compressor pressure, a LED head 3 for light therapy, a head for endermomassage 4a with a diameter of at least 60 mm and a head 4b with a diameter of at least 100 mm, a head 5 for oxygen infusion, that is oxybrasion, a head 6 for nano-needle mesotherapy, a head 7 for diamond microdermabrasion and a head 8 for oxygen infusion. The device is controlled by means of the base station 1 equipped with a touch screen 1a, and software and algorithms for treatment programmes or single treatments for a face, a neck, a cleavage, arms, a stomach, bottoms, thighs, palms, feet and a head skin with a "step by step" guiding mode, which after creating a USER PROFILE 21 and entering CONTRAINDICATIONS 9 by means of information presented in the form of a text and animation, provides instructions which enable the user, staying at home, to perform independently only the treatments or treatment programmes which are safe for such a user.

Guiding treatments or treatment programmes by means of a multifunctional touch screen of the cosmetic device is characteristic for the fact that after creating and saving a USER PROFILE 21 and entering CONTRAINDICATIONS 9, a SELECTION SCREEN 10 is displayed with an option of choosing one of three operating modes: TREATMENTS 11 with access to possible single treatments for a given part of the body, which take into account CONTRAINDICATIONS 9; PROGRAMMES 12 with access to possible treatment programmes for a given part of the body which take into account CONTRAINDICATIONS 9; and PRO 13 with access to all the possible treatments but with indication for use by professionals. Moreover, the device has a system of MESSAGES AND WARNINGS 15 in case a user wants to undergo treatments advised against or treatments in which a user selects excessive time or increased dose. In addition, after each finished treatment, the device displays RECOMMENDATIONS 16 of behaviours after a given treatment and suggestions of transferring some treatments to a group of preferred treatments, that is FAVOURITES 17.

A manner of guiding a treatment or treatment programmes after selecting the FACE icon 14 in the PROGRAMMES operating mode 12 displays a possibility of choosing various treatment programmes 14a; whereas, after choosing one of them, for instance, SCARS 18, the device proposes a diamond Microdermabrasion and/or nano-needle Mesotherapy and/or orange LED therapy, then a board 19 is displayed with products and heads necessary for performing the treatment, after selecting the diamond Microdermabrasion treatment, on the subsequent board, the device informs about ingredients which should be found in a preparation for the diamond Microdermabrasion, and the next board 22 provides the general information to the user about the DIAMOND MICRODERMABRASION. Then, in the form of a short animated film and a description, the device demonstrates subsequent activities which must be done to prepare your skin for the treatment, to prepare the device for work and it shows how to apply the head and how to move it on the skin in order to perform the treatment effectively and correctly, and afterwards, how to dismantle and clean the head so that it is ready for work next time; and after finishing the treatment and cleaning the head, the device presents RECOMMENDATIONS 16 of behaviours after the treatment.

A manner of guiding treatments or treatment programmes after selecting the FACE icon 14 in the PROGRAMMES operating mode 12, a possibility of choosing various treatment programmes 14a is displayed, and after choosing one of them, for instance, SCARS 18, the device proposes a Microdermabrasion and/or nano-needle Mesotherapy and/or orange LED treatment, then a board 19 is displayed with products and heads necessary for performing the treatment. After selecting the nano-needle Mesotherapy treatment and displaying general information 56 about this type of treatment, on the subsequent board, the device informs about the way of cleaning and preparing your face skin for the treatment, and afterwards the device instructs to apply a preparation for the nano-needle mesotherapy and specifies ingredients which should be found in a preparation for Mesotherapy. Then, the device instructs to wear protective gloves and, by means of a short animated film and a description, it demonstrates subsequent activities which must be done to prepare the device for work by selecting a proper cartridge, and it informs that the mesotherapy head has an option of adjusting the extension slide, suggesting a proper extension slide for respective parts of the face; the device informs how to set a proper extension slide and how to apply the head, and how to move it on the skin in order to perform the treatment effectively and correctly, and afterwards, how to dismantle and clean the head so that it is ready for work next time. After finishing the treatment and cleaning the head, the device presents RECOMMENDATIONS 16 of behaviours after the treatment.

A manner of guiding treatments or treatment programmes, after selecting the FACE icon 14 in the PROGRAMMES operating mode 12, a possibility of choosing various treatment programmes 14a is displayed, and after choosing one of them, for instance, SCARS 18, the device proposes a Microdermabrasion and/or nano-needle Mesotherapy and/or orange LED treatment, then a board 19 is displayed with products and heads necessary for performing the treatment. After selecting the orange LED treatment and displaying general information 90 about this type of treatment, on the subsequent boards, the device informs about the way of cleaning and preparing your face skin for the treatment, then, the device informs how to prepare it and how to move the head on the skin in order to perform the treatment effectively and correctly. Afterwards, it instructs to use a moisturising mask on the face and how to dismantle and clean the head so that it is ready for work next time. After finishing the treatment and cleaning the head, the device presents RECOMMENDATIONS 16 of behaviours after the treatment.

## Claims

1. A multifunctional cosmetic device for rejuvenating and body shaping treatments, **wherein,** it is characteristic for its compact size in relation to large professional devices and intended for use at home. It consists of
a base station (1) for controlling, connecting and starting respective heads,
a shockwave compressor head (2) for body shaping and firming, which operates under the compressor pressure,
a LED head (3) for light therapy,
a head (4a) with a diameter of at least 50 mm and a head (4b) with a diameter of at least 90 mm for endermomassage,
a head (5) for oxygen infusion, that is oxybrasion,
a head (6) for nano-needle mesotherapy,
a head (7) for diamond microdermabrasion and
a head (8) for oxygen infusion,
whereas, the device is controlled by means of the base station (1) equipped with a touch screen (1a), and software and algorithms for treatment programmes or single treatments for a face, a neck, a cleavage, arms, a stomach, bottoms, thighs, palms, feet and a head skin with a "step by step" guiding mode, which after creating a USER PROFILE 21 and entering CONTRAINDICATIONS 9 by means of information presented in the form of a text and animation, provides instructions which enable the user, staying at home, to perform independently only the treatments or treatment programmes which are safe for such a user.

2. Guiding treatments or treatment programmes by means of a multifunctional touch screen of the cosmetic device, **wherein,** after creating and saving a USER PROFILE 21 and entering CONTRAINDICATIONS 9, a SELECTION SCREEN 10 is displayed with an option of choosing one of three operating modes: TREATMENTS 11 with access to possible single treatments for a given part of the body, which take into account CONTRAINDICATIONS 9; PROGRAMMES 12 with access to possible treatment programmes for a given part of the body which take into account CONTRAINDICATIONS 9; and PRO 13 with access to all the possible treatments but with indication for use by professionals; moreover, the device has a system of MESSAGES AND WARNINGS 15 in case a user wants to undergo treatments advised against or treatments in which a user selects excessive time or increased dose; in addition, after each finished treatment, the device presents RECOMMENDATIONS 16 of behaviours after a given treatment and suggestions of transferring some treatments to a group of preferred treatments, that is FAVOURITES 17.

3. Guiding treatments or treatment programmes according to reservations 2, **wherein,** after selecting the FACE icon 14 in the PROGRAMMES operating mode 12, a possibility of choosing various treatment programmes 14a is displayed, and after choosing one of them, for instance, SCARS 18, the device proposes a diamond Microdermabrasion and/or nano-needle Mesotherapy and/or orange LED therapy, then a board 19 is displayed with products and heads necessary for performing the treatment; after selecting the diamond Microdermabrasion treatment, on the subsequent board 20, the device informs about ingredients which should be found in a preparation for the diamond Microdermabrasion, and the next board 22 provides the user with the general information about the DIAMOND MICRODERMABRASION, then, in the form of a short animated film and a description, the device demonstrates subsequent activities which must be done to prepare your skin for the treatment, to prepare the device for work and it shows how to apply the head and how to move it on the skin in order to perform the treatment effectively and correctly, and afterwards, how to dismantle and clean the head so that it is ready for work next time; and after finishing the treatment and cleaning the head, the device presents the RECOMMENDATIONS 16 of behaviours after the treatment.

4. Guiding treatments or treatment programmes according to reservations 2, **wherein,** after selecting the FACE icon 14 in the PROGRAMMES operating mode 12, a possibility of choosing various treatment programmes 14a is displayed, and after choosing one of them, for instance, SCARS 18, the device proposes a diamond Microdermabrasion and/or nano-needle Mesotherapy and/or orange LED therapy, then a board 19 is displayed with products and heads necessary for performing the treatment; after selecting the nano-needle Mesotherapy treatment, and displaying general information about this type of treatment, on the subsequent board 56, the device informs about the way of cleaning and preparing your face skin for the treatment, and afterwards the device instructs to apply a preparation for the nano-needle mesotherapy and instructs to wear protective gloves and, by means of a short animated film and a description, it demonstrates subsequent activities which must be done to prepare the device for work by selecting a proper cartridge, and it informs that the mesotherapy head has an option of adjusting the extension slide, suggesting a proper extension slide for respective parts of the face, the device informs how to set a proper extension slide and how to apply the head, and how to move it on the skin in order to perform the treatment effectively and correctly, and afterwards, how to dismantle and clean the head so that it is ready for work next time, and after finishing the treatment and cleaning the head, the device presents RECOMMENDATIONS 16 of behaviours after the treatment.

5. Guiding treatments or treatment programmes according to reservations 2, **wherein,** after selecting the FACE icon 14 in the PROGRAMMES operating mode 12, a possibility of choosing various treatment programmes 14a is displayed, and after choosing one of them, for instance, SCARS 18, the device proposes a Microdermabrasion and/or nano-needle Mesotherapy and/or orange LED therapy, then a board 19 is displayed with products and heads necessary for performing the treatment; after selecting the orange LED treatment and displaying the general information about the treatment on board 90, on the subsequent board the device informs about the way of cleaning and preparing your face skin for the treatment, and then it informs how to prepare the device and how to move the head on the skin in order to perform the treatment effectively and correctly, and afterwards, it instructs to use a moisturising mask on the face and how to dismantle and clean the head so that it is ready for work next time, and after finishing the treatment and cleaning the head, it displays the RECOMMENDATIONS 16 for behaviours after the treatment and the device deactivation screen 102.
